**Europäisches Patentamt**

**European Patent Office**

**Office européen des brevets**

(19)

(11) Veröffentlichungsnummer : **0 313 602 B1**

(12) **EUROPÄISCHE PATENTSCHRIFT**

(45) Veröffentlichungstag der Patentschrift :
22.04.92 Patentblatt 92/17

(51) Int. Cl.$^5$ : **A61M 25/00**

(21) Anmeldenummer : **88903776.8**

(22) Anmeldetag : **10.05.88**

(86) Internationale Anmeldenummer :
**PCT/DE88/00279**

(87) Internationale Veröffentlichungsnummer :
**WO 88/08726 17.11.88 Gazette 88/25**

(54) **VORRICHTUNG ZUR ENDOSKOPISCH-TRANSPAPILLÄREN SONDIERUNG EINES GALLENWEGESYSTEMS.**

(30) Priorität : **12.05.87 DE 3715699**

(43) Veröffentlichungstag der Anmeldung :
**03.05.89 Patentblatt 89/18**

(45) Bekanntmachung des Hinweises auf die
Patenterteilung :
**22.04.92 Patentblatt 92/17**

(84) Benannte Vertragsstaaten :
**AT BE CH DE FR GB IT LI LU NL SE**

(56) Entgegenhaltungen :
**EP-A- 0 132 215**
**DE-A- 2 923 633**
**DE-A- 3 004 335**
**DE-C- 3 447 642**
**US-A- 3 973 556**
**US-A- 4 033 331**
**US-A- 4 253 467**

(73) Patentinhaber : **FOERSTER, Ernst**
**Lachnerstrasse 71**
**W-8520 Erlangen (DE)**
Patentinhaber : **DOMSCHKE, Wolfram**
**Ebradstrasse 1**
**W-8520 Erlangen (DE)**

(72) Erfinder : **FOERSTER, Ernst**
**Lachnerstrasse 71**
**W-8520 Erlangen (DE)**
Erfinder : **DOMSCHKE, Wolfram**
**Ebradstrasse 1**
**W-8520 Erlangen (DE)**

(74) Vertreter : **Böhme, Volker, Dipl.-Ing.**
**Patentanwälte Dipl.-Ing. E. Kessel Dipl.Ing. V.**
**Böhme Karolinenstrasse 27**
**W-8500 Nürnberg 1 (DE)**

EP 0 313 602 B1

## Beschreibung

Die Erfindung betrifft eine Vorrichtung zur endoskopisch-transpapillären Sondierung eines Gallenwegesystems nach dem Oberbegriff des Anspruchs 1.

Eine bekannte (DE-PS 34 47 642) Vorrichtung dieser Art umfaßt nur das Seitblick-Duodenoskop, den Sondierungskatheter und den Führungsdraht und läßt nur die Untersuchung des Hauptgallenganges zu. Es besteht aber das Problem der sicheren und reproduzierbaren Darstellung der menschlichen Gallenblase auf endoskopisch-transpapillärem Wege. Es ist zwar die perkutane-transhepatische Punktion und Drainage der Gallenblase bekannt. Dieses Verfahren hat den Nachteil, daß ein künstlicher Zugang durch die Haut eröffnet werden muß, das Risiko einer Gewebsverletzung recht groß ist und die Belastung für den Patienten entsprechend problematisch ist.

Eine Aufgabe der Erfindung ist es daher, eine Vorrichtung der eingangs genannten Art zu schaffen, die eine sichere, gezielte und reproduzierbare Darstellung des Gallenblasenlumens auf endoskopisch-retrogradem Wege ermöglicht. Die erfindungsgemäße Vorrichtung ist, diese Aufgabe lösend, dadurch gekennzeichnet, daß der Sondierungskatheter und der Führungsdraht zum Vorschieben bis zum Ductus cysticus vorgesehen sind ist, daß ein Führungskatheter zum Schieben über den Führungsdraht vorgesehen ist, entsprechend dem Judkins- oder Rechtskoronar-Katheter mit Biegungen präformiert ist und eine konisch geformte Spitze aufweist, die bei zurückgezogenem Führungsdraht in den Ductus cysticus geschwenkt ist, daß ein Sondierungsdraht mit flexiblem Spitzenstück zum Schieben durch den Führungskatheter und durch den Ductus cysticus in das Gallenblasenlumen vorgeschen ist und daß bei zurückgezogenem Führungskatheter ein Spülkatheter oder ein kleines Endoskop zum Schieben mittels des Sondierungsdrahtes in das Gallenblasenlumen vorgesehen ist.

Mit der erfindungsgemäßen Vorrichtung gelingt eine sichere Sondierung des Ductus cysticus, des Ausführungsganges der Gallenblase, obwohl der Ductus cysticus mit einem Durchmesser von ca. 1 - 2 mm schwer zu finden ist, darüber hinaus spiralförmig konfiguriert ist und durch mehrere in Richtung des Gallenblasenlumens geöffnete Klappen, die sog. Heisterschen Klappen, verengt ist. Die Vorrichtung gewährleistet eine sichere, gezielte Manipulation zur Sondierung des Ductus cysticus. Die Teile der Vorrichtung werden durch ein handelsübliches Seitblick-Duodenoskop eingeführt.

Die Abmessungen der Präformierung des Führungskatheters entsprechen den Abzweigungen des Gallenwegesystems. Der Sondierungsdraht ist an dem Vorderteil biegeweich und im Rest steif und biegesteif ausgebildet. Die Biegsamkeit dient der Umgehung von Widerständen. Der Ballon des Spülkatheters dient zum Verschließen der Gallenblase und das Spülen zur endoskopisch-transpapillären Lyse von Gallensteinen.

Besonders zweckmäßig und vorteilhaft ist, wenn der Spülkatheter mehrere Kanäle zum Spülen, Absaugen und Aufblasen aufweist. Dies erleichtert die Bedienung des Spülkatheters.

Besonders zweckmäßig und vorteilhaft ist es auch, wenn das kleine Endoskop mit einer Führungsschiene oder einem Arbeitskanal zum Aufschieben auf den Sondierungsdraht versehen ist. Dies erleichtert die Arbeitsweise. Es ist allerdings auch möglich, über den Sondierungsdraht einen Hilfskatheter zu schieben und nach Zurückziehung des Sondierungsdrahtes das kleine Endoskop durch den Hilfskatheter zu schieben.

Besonders zweckmäßig und vorteilhaft ist es auch, wenn das flexible Spitzenstück von Spiraldraht gebildet ist und außen mit einem glatten Kunststoffüberzug versehen ist. Bei Biegungen des Spiraldrahtstückes kann wegen des Kunststoffüberzuges zwischen Drahtwindungen Gewebe nicht eingeklemmt werden.

Besonders zweckmäßig und vorteilhaft ist es ebenso, wenn das vordere Ende des Sondierungsdrahtes mit einem glatten Rundstück versehen ist. Diese sog. Kaltenbach-Spitze ist ein einfaches Mittel, um das vordere Ende des Sondierungsdrahtes abgerundet glatt zu gestalten.

Mit der erfindungsgemäßen Vorrichtung wird ein Verfahren zur endoskopisch-transpapillären Sondierung der Gallenblase durchgeführt,

bei dem ein Seitblick-Duodenoskop in den Zwölffinderdarm, endend bei der Duodenalpapille, eingeführt wird,

bei dem ein Sondierungskatheter durch das Seitblick-Duodenoskop und die Duodenalpapille in den Hauptgallengang bis zur Mündung des Ductus cysticus geschoben wird,

bei dem durch den Sondierungskatheter ein Führungsdraht vorgeschoben wird und dann der Sondierungskatheter zurückgezogen wird, bei dem ein Führungskatheter über den Führungsdraht geschoben wird, entsprechend dem Judkins- oder Rechtskoronar- Katheter präformiert ist, in den Abmessungen den Abzweigungen der Gallenblase entspricht und eine konisch geformte Spitze aufweist,

bei dem nach Zurückziehen des Führungsdrahtes der Führungskatheter aufgrund der Präformierung mit der konischen Spitze in den Ductus cysticus schwenkt,

bei dem ein Sondierungsdraht durch den Führungskatheter und durch den Ductus cysticus in die Gallenblase schiebbar ist und bei dem mittels des Sondierungsdrahtes ein Werkzeug in die Gallenblase eingeschoben und danach der Sondierungsdraht zurückgezogen wird.

In der Zeichnung ist eine bevorzugte Ausführungsform der Erfindung dargestellt und zwar eine Vorrichtung zur endoskopisch-transpapillären Sondierung eines Gallenwegesystems. Es zeigt

Fig. 1 ein Seitblick-Duodenoskop der Vorrichtung im Gallenwegesystem,

Fig. 2 das Seitblick-Duodenoskop gemäß Fig. 1 in einem gegenüber Fig. 1 vergrößerten Maßstab,

Fig. 3 einen Sondierungskatheter im Gallenwegesystem,

Fig. 4 einen Führungsdraht im Gallenwegesystem,

Fig. 5 einen Führungskatheter im Gallenwegesystem mit teilweise zurückgezogenem Führungsdraht,

Fig. 6 die Spitze des Führungskatheters in einem gegenüber Fig. 5 vergrößerten Maßstab,

Fig. 7 den Führungskatheter gemäß Fig. 5 bei vollständig zurückgezogenem Führungsdraht,

Fig. 8 einen Sondierungsdrahf im Gallenwegesystem, durch den Führungskatheter geschoben,

Fig. 9 den Sondierungsdraht gemäß Fig. 8 bei zurückgezogenem Führungsdraht,

Fig. 10 eine Einzelheit der Fig. 9 in einem gegenüber Fig. 9 vergrößerten Maßstab,

Fig. 11 einen Spülkatheter im Gallenwegesystem bei zurückgezogenem Führungsdraht,

Fig. 12 ein kleines Endoskop im Gallenwegesystem bei zurückgezogenem Führungsdraht,

Fig. 13 im Schnitt ein Teil des kleinen Endoskops in einem gegenüber Fig. 12 vergrößerten Maßstab,

Fig. 14 das vordere Endstück des Sondierungsdrahtes in einem gegenüber Fig. 8 vergrößerten Maßstab,

Fig. 15 einen Judkinskatheter,

Fig. 16 einen Katheter mit abgebogenem Endstück und

Fig. 17 den Spülkatheter in einem gegenüber Fig. 11 vergrößerten Maßstab.

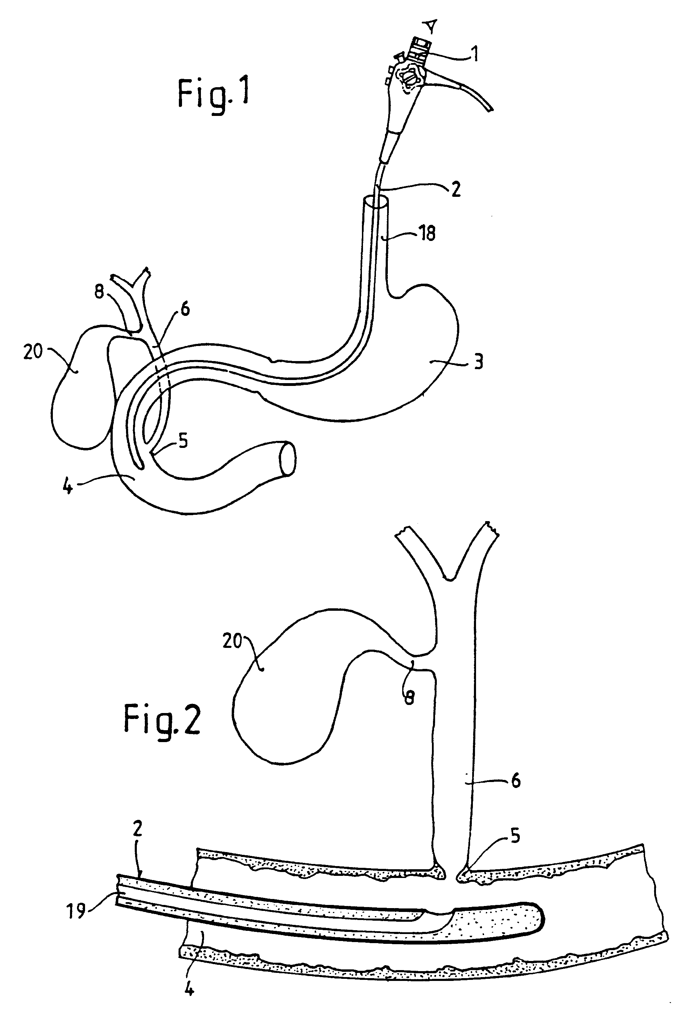

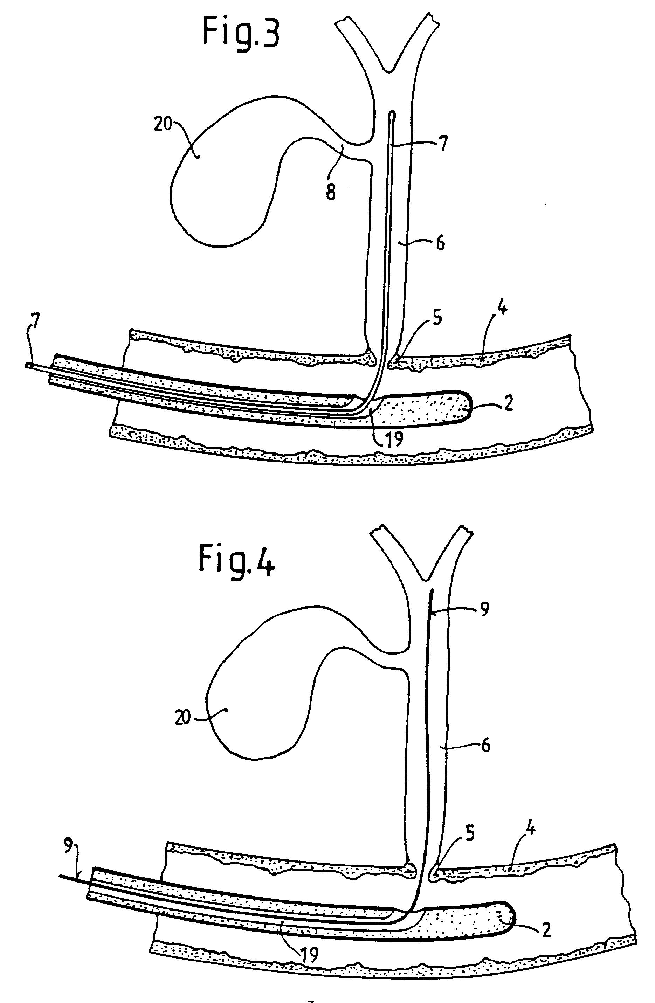

Gemäß Fig. 1 und 2 ist ein mit einer Einblickeinrichtung 1 versehenes Seitblick-Duodenoskop 2 vorgesehen, das durch eine Speiseröhre 18 und einen Magen 3 in einen Zwölffingerdarm 4 geschoben ist und zwar bis zur Duodenalpapille 5. Fig. 1 und 2 zeigen also die antomischen Verhältnisse bei eingeführtem Seitblick-Duodenoskop 2, das einen Arbeitskanal 19 aufweist. An der Duodenalpapille 5 zweigt der Hauptgallenweg 6 ab, in den gemäß Fig. 3 ein Sondierungskatheter 7 über den Arbeitskanal 19 eingeführt ist. über den Arbeits- bzw. Instrumentierkanal 19 des durch die Magen-Darmpassage zur Duodenalpapille 5 eingeführten Seitblick-Duodenoskops 2 wird also in den Hauptgallengang 6 der Sondierungskatheter 7 eingeführt, der eine Kontrastmitteldarstellung und eine Röntgenkontrolle der intra- wie extrahepatitischen Gallenwege erlaubt. Der Sondierungskatheter 7 ist ähnlich aufgebaut wie ein gebräuchlicher Teflon-Katheter zur Sondierung des Hauptgallenganges.

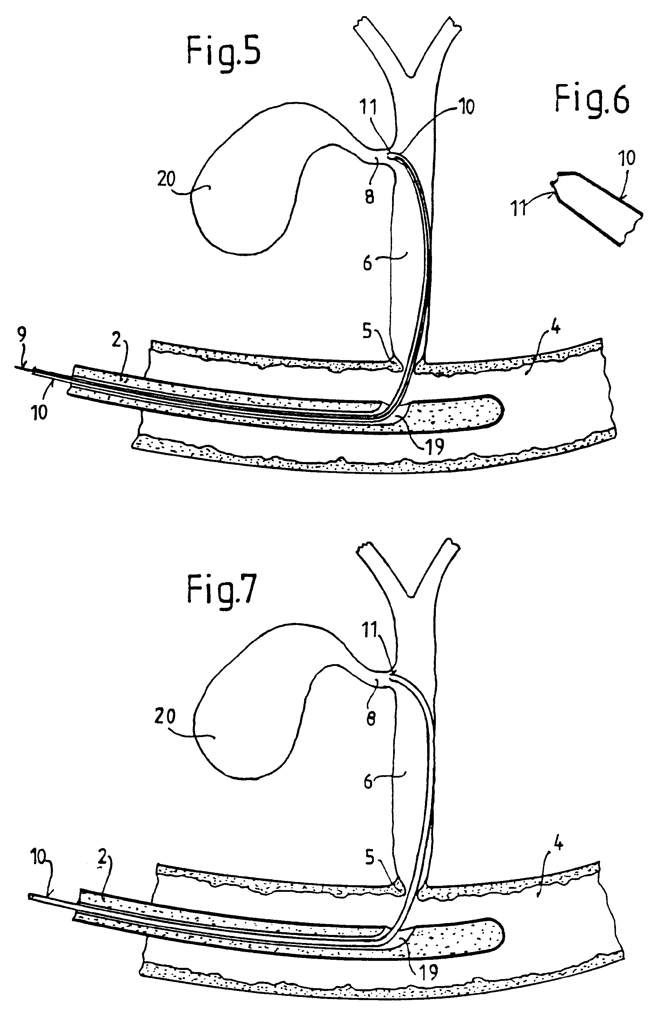

Von dem Hauptgallengang 6 zweigt ein Ductus cysticus 8 ab, der in ein Gallenblasenlumen 20 übergeht. Der Sondierungskatheter 7 ist mit dem freien Ende etwas am Ductus cysticus 8 vorbeigeschoben. Es wird ein Führungsdraht 9 durch den Sondierungskatheter 7 geschoben, der sodann zurückgezogen bzw. entfernt wird, wonach der Führungsdraht 9 gemäß Fig. 4 in den Hauptgallengang 6 eingeführt ist. Der Führungsdraht 9 ist ähnlich aufgebaut wie ein gebräuchlicher Metall-Spiraldraht zur Sondierung des Hauptgallenganges. Es wird nun gemäß Fig. 5 ein Führungskatheter 10 durch den Arbeitskanal 19 über den Führungsdraht 9 in den Hauptgallengang 6 eingeführt. Gemäß Fig. 5 ist der Führungsdraht 9 partiell zurückgezogen. Der Führungskatheter 10 weist gemäß Fig. 6 eine konische Spitze 11 auf. Gemäß Fig. 7 ist der Führungsdraht 9 vollständig zurückgezogen und okkludiert der Führungskatheter 10 mit der konischen Spitze 11 die Mündung des Ductus cysticus 8 in den Hauptgallengang 6.

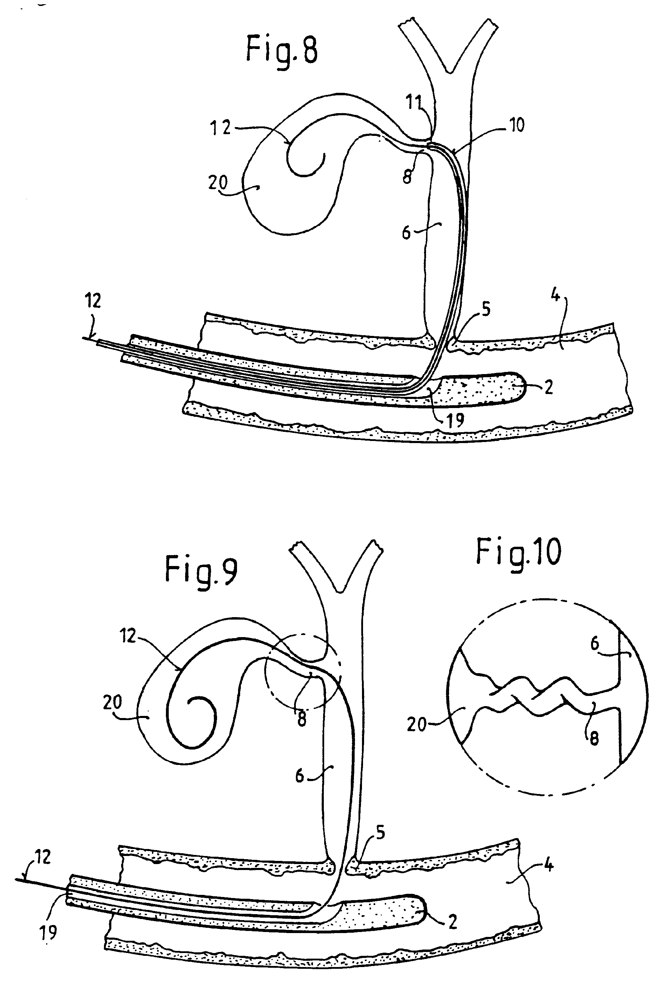

Gemäß Fig. 8 wird durch den in den Hauptgallengang 6 eingeführten Führungskatheter 10 ein Sondierungsdraht 12 vorgeschoben und zwar in den Ductus cysticus 8 und in das Gallenblasenlumen 20. Der Führungskatheter 10 wird sodann zurückgezogen und entfernt, wobei der Sondierungdraht im Gallenblasenlumen 20 belassen wird, wie es in Fig. 9 gezeigt ist. Fig. 10 zeigt die detaillierten anatomischen Verhältnisse am Ductus cysticus 8, der üblicherweise eine spiralförmige Konfiguration besitzt und die Heisterschen Klappen 21 aufweist.

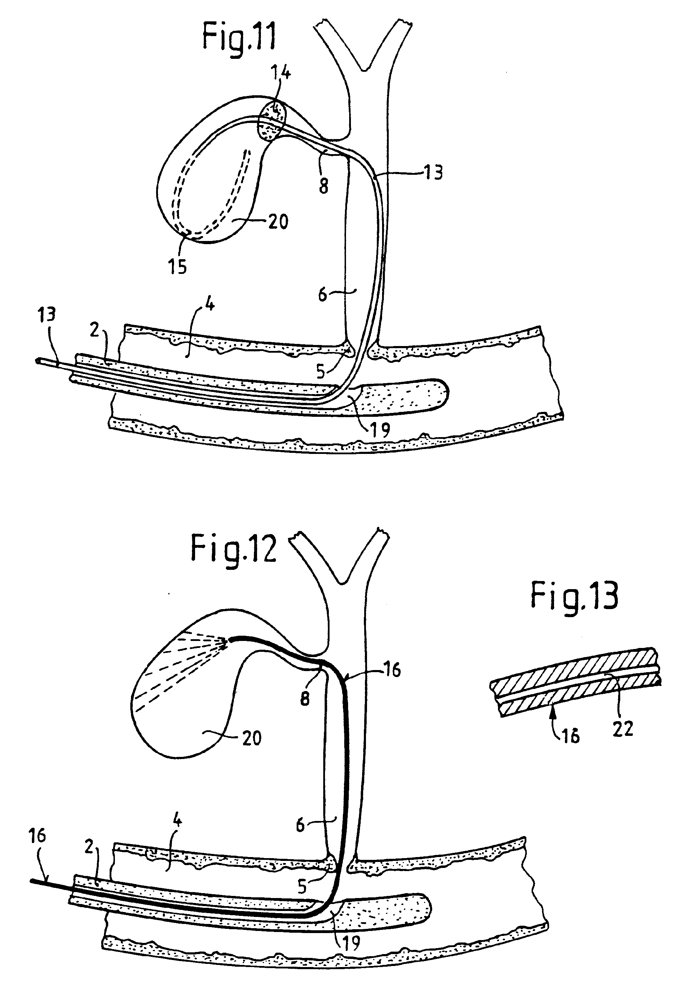

Gemäß Fig. 11 ist ein Spülkatheter 13 in das Gallenblasenlumen 20 vorgeschoben. Der Spülkatheter 13 wird in das Gallenblasenlumen 20 über den Sondierungsdraht 12 vorgeschoben, der anschließend entfernt wird. Wahlweise wird gemäß Fig. 12 über den Sondierungsdraht 12 ein kleines Endoskop 16 in das Gallenblasenlumen 20 vorgeschoben und wird anschließend der Sondierungsdraht 20 entfernt. Das kleine Endoskop 16 weist eine üblicherweise verwendete optische Einrichtung und gemäß Fig. 13 einen führenden Arbeitskanal 22 auf, der den Sondierungsdraht 12 aufnimmt.

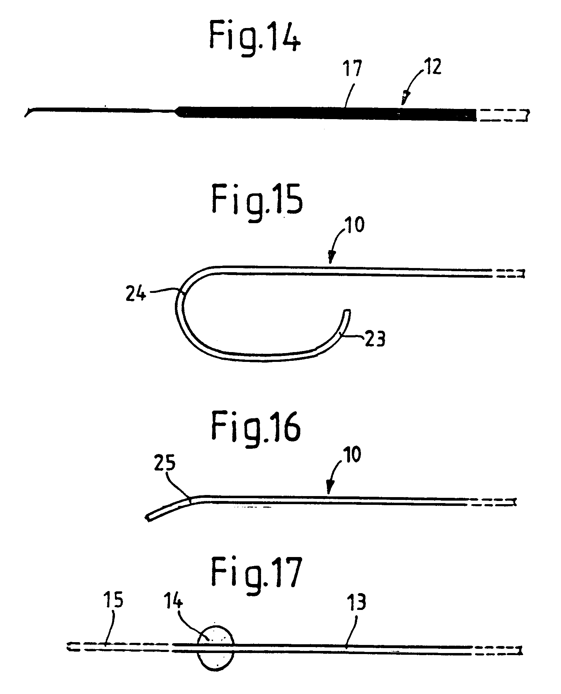

In Fig. 14 ist der Sondierungsdraht 12 dargestellt, der das flexible Spitzenstück 17 aufweist. Der Sondierungsdraht 12 ist ähnlich aufgebaut wie ein gebräuchlicher Metall-Spiraldraht zur Sondierung der Koronargefäße. Die Länge des flexiblen Spitzenstückes 17 ist den Dimensionen des Hauptgallenganges 6 und des Ductus cysticus 8 angepaßt. Die Länge des Sondierungsdrahtes 12 ist dem Seitblick-Duodenoskop 2 angepaßt. Der Führungskatheter 10 nach Fig. 15 ist ähnlich aufgebaut wie ein gebräuchlicher Führungskatheter zur Sondierung des linken Koronarostiums und weist eine dem Seitblick-Duodenoskop angepaßte Länge auf. Der Führungskatheter 10 ist wie ein üblicher Judkinskatheter ausgebildet und besitzt präformierte Biegungen 23, 24, die dem Abstand zwischen der Duodenalpapille 5 und der Mündung des Ductus cysticus 8 angepaßt sind.

Fig. 16 zeigt einen Führungskatheter 10 in der Form eines üblicherweise zur Sondierung des rechten Koronarostiums verwendeten Rechtskoronar-Katheters, wobei eine vorgeformte Biegung 25 dem Abstand zwischen der Duodenalpapille 5 und der Mündung des Ductus cysticus 8 angepaßt ist und die Länge dem Seitblick-Duodenoskop angepaßt ist. Der Spülkatheter 13 gemäß Fig. 17 trägt einen 5 - 10 mm im Durchmesser messenden aufblasbaren Ballon 14 und ein 10 - 20 cm messendes Katheterspülspitzenstück mit multiplen, seitlich angebrachten Öffnung 15 zum Spülen und Absaugen. Die Länge des Spülkatheters 13 ist dem Seitblick-Duodenoskop 2 angepaßt.

## Patentansprüche

1. Vorrichtung zur endoskopisch-transpapillären Sondierung eines Gallenwegesystems, bei der ein Seitblick-Duodenoskop (2) zum Einführen in den Zwölffingerdarm (4), endend bei der Duodenalpapille (5) vorgesehen ist, bei der ein Sondierungskatheter (7) durch das Seitblick-Duodenoskop (2) und die Duodenalpapille (5) in den Hauptgallengang (6) schiebbar ist und bei der ein Führungsdraht (9) zur Anordnung in dem Seitblick-Duodenoskop (2) und dem Sondierungskatheter (7) vorgesehen ist, **dadurch gekennzeichnet, daß** der Sondierüngskatheter (7) und der Führungsdraht (9) zum Vorschieben bis zum Ductus cysticus (8) vorgesehen sind, daß ein Führungskatheter (10) zum Schieben über den Führungsdraht (9) vorgesehen ist, entsprechend dem Judkinsoder dem Rechtskoronar-Katheter mit Biegungen präformiert ist und eine konische geformte Spitze (11) aufweist, die bei zurückgezogenem Führungsdraht (9) in den Ductus cysticus (8) geschwenkt ist, daß ein Sondierungsdraht (12) mit flexiblem Spitzenstück (17) zum Schieben durch den Führungskatheter (10) und durch den Ductus cysticus (8) in das Gallenblasenlumen (20) vorgesehen ist und daß bei zurückgezogenem Führungskatheter (10) ein Spühlkatheter (13) oder ein kleines Endoskop (16) zum Schieben mittels des Sondierungsdrahtes (12) in das Gallenblasenlumen (20) vorgesehen ist.

2. Vorrichtung nach Anspruch 1, **dadurch gekennzeichnet, daß** der Spühlkatheter (13) mehrere Kanäle zum Spülen, Absaugen und Aufblasen aufweist.

3. Vorrichtung nach Anspruch 1, **dadurch gekennzeichnet, daß** das kleine Endoskop (16) mit einer Führungsschiene oder einem Arbeitskanal zum Aufschieben auf den Sondierungsdraht (12) versehen ist.

4. Vorrichtung nach Anspruch 1, 2 oder 3, **dadurch gekennzeichnet, daß** das flexible Spitzenstück (17) von Spiraldraht gebildet ist und außen mit einem glatten Kunststoffüberzug versehen ist.

5. Vorrichtung nach einem der vorhergehenden Ansprüche, **dadurch gekennzeichnet, daß** das vordere Ende des Sondierungsdrahtes mit einem glatten Rundstück versehen ist.

## Claims

1. Device for endoscopic transpapillary probing of a biliary tract system, in which a lateral vision duodenoscope for insertion into the duodenum, ending at the duodenal papilla, is provided, in which a probing catheter can be pushed through the lateral vision duodenoscope and the duodenal papilla into the choledochus, and in which a guide wire is provided for positioning in the lateral vision duodenoscope and the probing catheter, characterized in that the probing catheter (7) and the guide wire (9) are suitably constructed for insertion up to the ductus cysticus (8), in that a guide catheter (10) is provided for pushing over the guide wire (9), is preformed with bends (23, 24, 25) corresponding to the Judkins or right coronary catheter and has a conically shaped tip (11) which is swivelled in the ductus cysticus (8) with the guide wire (9) withdrawn, in that a probing wire (12) with a flexible tip-piece (17) can be pushed through the guide catheter (10) and through the ductus cystious (8) into the gallbladder lumen (20), and in that a flushing catheter (13) or a small endoscope (16) can be pushed into the gallbladder lumen (20) by means of the probing wire (12) with the guide catheter (10) withdrawn.

2. Device according to Claim 1, characterized in that the flushing catheter (13) has several channels for flushing, suction and inflation.

3. Device according to Claim 1 or 2, characterized in that the small endoscope (16) is provided with a guide track or a working channel (22) for pushing onto the probing wire (12).

4. Device according to Claim 1, 2 or 3, characterized in that the flexible tip-piece (17) is formed by spiral wire and is provided on the outside with a smooth covering of plastic.

5. Device according to one of the preceding claims, characterized in that the front end of the probing wire is provided with a smooth round piece.

## Revendications

1. Dispositif d'exploration endoscopique transpapillaire des voies biliaires, dans lequel il est prévu un duodénoscope à vision latérale pour introduction dans le duodénale, aboutissant au niveau de la papille duodénale, dans lequel un cathéter d'exploration peut être introduit par le duodénoscope à vision latérale et la papille duodénale dans le canal cholédoque et dans lequel il est prévu un fil de guidage à mettre en place dans le duodénoscope à vision latérale et le cathéter d'exploration, caractérisé en ce que le cathéter d'exploration (7) et le fil de guidage (9) ont une configuration appropriée pour être introduits jusqu'au canal cystique (8), qu'il est prévu pour l'introduction sur le fil de guidage (9) un cathéter de guidage (10) correspondant au cathéter de Judkins ou au cathéter coronaire droit, préformé par des courbures (23, 24, 25) et possédant une pointe conique (11) qui bascule dans le canal cystique (8) lorsqu'est retiré le fil de guidage (9), qu'un fil d'exploration (12) à embout (17) souple peut être introduit dans la lumière (20) de la vésicule biliaire par le cathéter de guidage (10) et le canal cystique (8) et qu'un cathéter de rinçage (13) ou un petit endoscope (16) peut être introduit dans la lumière (20) de la vésicule biliaire au moyen du fil d'exploration (12) lorsqu'est retiré le cathéter de guidage (10).

2. Dispositif selon la revendication 1, caractérisé en ce que le cathéter de rinçage (13) présente plusieurs canaux destinés au rinçage, à l'aspiration et au gonflement.

3. Dispositif selon la revendication 1 ou 2, caractérisé en ce que le petit endoscope (16) est muni d'un rail de guidage ou d'un canal opérationnel (22) à enfiler sur le fil d'exploration (12).

4. Dispositif selon la revendication 1, 2 ou 3, caractérisé en ce que l'embout 17 souple est en un fil en spirale et est muni extérieurement d'un revêtement lisse en matière plastique.

5. Dispositif selon l'une des revendications précédentes, caractérisé en ce que l'extrémité avant du fil d'exploration est munie d'un embout rond lisse.

Fig.1

Fig.2

# Fig.3

# Fig.4

Fig.5

Fig.6

Fig.7

Fig. 8

Fig. 9

Fig. 10

Fig.11

Fig.12

Fig.13

Fig.14

Fig.15

Fig.16

Fig.17